# EUROPEAN PATENT APPLICATION

(11) **EP 2 775 413 A2**
(43) Date of publication of application: **10.09.2014**
(21) Application number: 13197337.2
(22) Date of filing: 16.12.2013
(51) Int. Cl.: G06F 19/00

(54) **Control system for modular imaging device**

(30) Priority: 31.12.2012 US 201213731164
(71) Applicant: Karl Storz Imaging Inc., Goleta, CA 93117 (US)
(72) Inventor: Lee, D. Holoien, Goleta, CA 93117 (US); Amling, Marc R, Goleta, CA 93117 (US); Ruiz, Angeli Mancuso, Goleta, CA 93117 (US); McCleary, Los Angeles, CA 90028 (US)
(74) Representative: Witte, Weller & Partner Patentanwälte mbB

(57) **Abstract**

A medical imaging system including a control module having a processor, at least one input module transmitting identifying information once connected to the control module, a display coupled to the control module for displaying image data received from the at least one input module, and a software executing on the processor for presenting icons on the display associated with the identifying information.

## Description

### FIELD OF THE INVENTION

The invention relates to a system for controlling a modular imaging device, and more specifically the invention relates to a configurable control system that automatically presents and updates information regarding controllability, settings and operation status of imaging devices and medical equipment connected thereto and allows a user to configure the system in accordance with the user's preferences.

### BACKGROUND OF THE INVENTION

A wide variety of operating room systems are known for performing both diagnostic and surgical procedures. The known systems allow a surgeon to perform a procedure with a wide variety of medical and operating room equipment. This equipment ranges from visualization devices, such as endoscopes, cameras, etc., and systems, to medical devices, such as tools for cutting, grasping, extracting, irrigating, etc., and other operating room equipment.

In particular, visualization devices are known that allow for imaging of an interior of an organ or joint while a surgeon is conducting a procedure. These visualization systems allow a surgeon to view, typically on a surgical monitor placed either in or adjacent to a sterile environment, a location inside the body where the procedure is being performed. Known systems further allow for the recording of still pictures and video recordings of the area and procedure. Not only the surgeon and those in the operating room are able to view the surgical site on the surgical monitor, but the systems further provide for the transfer of visualization information via a network connection to remote locations from the operating room. In this manner, individuals have the ability to view a surgical procedure from different locations. This has proved to be a very helpful educational tool (e.g. medical students can view a medical procedure from a class room) and has allowed for specialists to view the surgical procedure from a distance to provide expert analysis and input to the surgeon.

In known medical imaging systems, endoscopic cameras are typically connected to a Camera Control Unit ("CCU"), with the CCU processing and displaying the imaging data transmitted from the endoscopic camera. Often, each medical procedure requires a different camera, leading to a large inventory of cameras. Additionally, each camera must be compatible with the CCU to function correctly. As such, each CCU has software to process and operate a variety of camera technologies, and as new technologies become available, the CCU may need updated software to properly process images from new camera technology. Additionally, the CCU hardware may become outdated, thus requiring an entirely new CCU to process the images of both old and new camera technologies used by a physician.

Traditionally, CCUs are compatible with a limited number of camera heads. A CCU's hardware is usually difficult to configure for proper communication with varying types of camera heads because camera heads use varying types of imaging devices that can differ in pixel resolution, timing requirements, signal output type, physical size, and in other characteristics. Additionally, there may be variability from device to device of the same type, which may affect camera head performance. Furthermore, commands sent from the CCU to the camera head are generally unique depending upon the camera head type being used. Moreover, as repairs, modifications, or improvements are made to camera heads, the CCU, which was originally designed to be compatible with the older camera head, may become incompatible and may require upgrading as well.

This overall variability in camera heads, either caused by imaging device technologies or by CCU command characteristics, often results in a CCU being specifically designed to be compatible with camera head type utilized. Also, consumers may desire different capabilities related to specific applications of the cameras, such medical, industrial, and scientific uses. Capabilities include picture to picture, reverse video, electronic zoom, still image capture, and stereoscopic video interface.

Moreover, CCUs are typically designed for use with camera head technologies currently in existence, and not designed to anticipate and accommodate camera heads yet to be developed. Hence, CCUs are typically not designed to be compatible with future camera head technologies; particularly, image device and image signal transmission technologies. These differences between older and newer camera heads also contribute to compatibility problems.

Because CCUs are usually compatible with limited quantities of camera heads, CCUs are typically discarded in favor of ones that were designed concurrently and/or to be compatible with particular camera head technologies. Consequently, CCUs have become an added expense often associated with changing imaging devices or camera heads. Further, it is typically desired for camera heads to be improved due to the demand from consumers to have the latest technology and advancement in equipment. Moreover, CCUs used in medical and veterinary fields are increasingly being mounted permanently in equipment bays or carts and/or permanently mounted within the walls of surgical operating rooms themselves. The expense associated with replacing CCUs to maintain compatibility with camera heads is subsequently passed onto consumers.

Control interfaces used with known medical imaging systems have also been in wide use in the industry for a number of years for control of imaging devices, including routing of medical visualization data. For example, U.S. Patent No. 8,069,420 to Plummer ("the '420 patent") discloses a system that allows for the identification of video collecting sources and video destinations such that a surgeon need only to select an icon on a touchscreen corresponding to the video input device and select another icon on the touchscreen corresponding to a desired destination, and the video is routed to the desired destination.

Other systems provide for integrated control systems that have limited control of medical equipment in an operating room. For example, U.S. Patent No. 5,788,688 to Bauer et al. ("the '688 patent") discloses a networked system for command and control of operating room equipment in the sterile environment. The '688 patent also discloses that surgeon's preset preferences may be uploaded such that the system may be pre-adjusted (within defined parameters) to surgeon's preferred settings thereby saving time and reducing possible errors in the adjustment and setting of equipment.

However, known control interfaces still suffer from a number of disadvantages. As more and more diverse imaging devices are introduced to the operating room that must be controlled by a surgeon and/or other medical staff, there is a need for control interfaces that are capable of integrating information about the variety of imaging devices connected to the control device. Each imaging device typically has specific and unique operating parameters that, if not operated uniformly and in concert with other imaging devices and equipment, can significantly impair surgeon's ability to perform a particular procedure and lead to negative consequences for a patient. Therefore, it is desirable to provide an imaging system control interface that allows for synchronized control of imaging devices and related medical equipment. It is further desirable to provide a control interface that automatically presents and updates information regarding controllability of imaging devices and medical equipment (that is, device/equipment presence on the system control bus), as well as the device and equipment settings and operational status.

Moreover, known user interfaces for endoscopic video systems are typically menu/text based schemes which require a user to step through menu trees to reach a desired setting to be changed or adjusted. Although intended to be intuitive, such schemes typically require a "learning period" during which users become accustomed to the layout and eventually memorize the menu tree for quick actuation of system controls and settings. However, as video camera systems become more complex and feature rich, and as more and more medical equipment is controlled via a menu/text based user interface, users are becoming inundated with control/command options and quickly succumb to information overload. Therefore, it is desirable to provide a user interface that is very intuitive and user friendly, and that is easily customizable in accordance with a surgeon's preference.

### SUMMARY OF THE INVENTION

The present invention is directed to solving one or more of the problems discussed above.

In accordance with aspects of the invention, a medical imaging system is provided including a control module having a processor, at least one input module transmitting identifying information once connected to the control module, a display coupled to the control module for displaying image data received from the at least one input module, and software executing on the processor for presenting icons on the display associated with the identifying information.

In some embodiments, the medical imaging system further includes at least one camera connected to the at least one input module. In certain of these embodiments, the at least one camera includes an endoscope. In additional of these embodiments, the at least one input module includes a processor for converting video received from the at least one camera into a format readable by the control module. In some of thede embodiments, the control module processor processes formatted video into at least one output video signal.

In certain embodiments, the display is a touchscreen displaying the icons and receiving actuation commands from a user.

In some cases, the identifying information presented on the display comprises a set of commands executable by the at least one input module.

In certain embodiments, the software is configurable such that the icons and the image data are displayed on the display in a manner selected by a user.

In some embodiments, upon selection of an input module icon by a user, the icon displays a plurality of available commands associated with the selected input module. In certain of these embodiments, the plurality of available commands are configured and presented on the display based upon a user's defined configuration.

In certain embodiments, the medical imaging system further includes a monitor connectable to the control module displaying the image data received from the at least one input module. In some of these embodiments, the software presents information associated with the monitor via the display. In additional of these embodiments, the image data is configured and presented on the monitor based upon a user's defined configuration.

In some cases, the medical imaging system further includes a storage device coupled to the control module, wherein the software presents information associated with the storage device via the display.

In certain embodiments, the at least one input module comprises a plurality of input modules, and each input module has an icon associated therewith that is presented by the software via the display. In some of these embodiments, the image data from the plurality of input modules is configured and presented on the display based upon a user's defined configuration.

In some advantageous embodiments, when an icon presented on the display is activated, the icon visibly changes so as to indicate to the user that the device associated with the icon is activated. In certain of these embodiments, the change to the icon comprises a change in the color of the icon. In other of these embodiments, the change to the icon comprises a change in the configuration of the icon. In additional of these embodiments, the change to the icon includes an alpha-numeric indication over the icon indicating a setting of the device associated with the icon. In yet further of these embodiments, when the icon is activated, a control interface is displayed on the display such that the user can change a setting of the device associated with the icon and upon changing the setting, the control interface is removed from the display and the changed setting is displayed on the icon associated with the device to which the setting was changed.

In certain embodiments, the medical imaging system also includes at least one medical tool coupled to the control module, wherein the software presents identifying information associated with the at least one medical tool via the display.

In some embodiments, the medical imaging system further includes at least one piece of operating room equipment coupled to the control module, wherein the at least one piece of operating room equipment has an icon associated therewith that is configured and presented on the display based upon a user's defined configuration.

In certain embodiments, the medical imaging system also includes an unlocking/locking mechanism presented on the display such that when the user activates the unlocking/locking mechanism, the display is selectively unlocked so that the user can manually adjust the positioning of the image data and icons on the display and upon activation of the unlocking/locking mechanism a second time, the position of the video output and icons on said display is locked. In some of these embodiments, a particular user's preferred configuration of the image data and icons on the display is stored and retrievable by that, or another, user.

In some cases, the display is a first display and the system further includes a second display positioned outside of the sterile environment, the second display coupled to the control module and providing all the functionality of the first display.

In certain embodiments, the control module is connectable to central operating room control system via a network connection. In some of these embodiments, the network connection is wireless.

Other objects of the invention and its particular features and advantages will become more apparent from consideration of the following drawings and accompanying detailed description.

### BRIEF DESCRIPTION OF THE DRAWINGS

FIG. 1 is a block diagram of an embodiment of a medical imaging system of the present invention.
FIG. 2 is a schematic illustration of a display of the medical imaging system of FIG. 1;
FIG. 3 is a schematic illustration of the display of FIG. 2, showing an icon selected.
FIG. 4 is a schematic illustration of the display of FIG. 2, showing another icon selected.
FIG. 5 is a schematic illustration of the display of FIG. 4, showing a change in the icon selected.
FIG. 6 is a schematic illustration of the display of FIG. 2.
FIG. 7 is a schematic illustration of the display of FIG. 6, showing one of the icons selected.
FIG. 8 is a schematic illustration of the display of FIG. 6, showing alternative configuration of icons.
FIG. 9 is a schematic illustration of the display of FIG. 6, showing alternative image configuration.

### DETAILED DESCRIPTION OF THE INVENTION

For this application the following terms and definitions shall apply:

The term "data" as used herein means any indicia, signals, marks, symbols, domains, symbol sets, representations, and any other physical form or forms representing information, whether permanent or temporary, whether visible, audible, acoustic, electric, magnetic, electromagnetic or otherwise manifested. The term "data" as used to represent predetermined information in one physical form shall be deemed to encompass any and all representations of the same predetermined information in a different physical form or forms.

The term "network" as used herein includes both networks and internetworks of all kinds, including the Internet, and is not limited to any particular network or inter-network.

The terms "first" and "second" are used to distinguish one element, set, data, object or thing from another, and are not used to designate relative position or arrangement in time.

The terms "coupled", "coupled to", "coupled with", "connected", "connected to", and "connected with" as used herein each mean a relationship between or among two or more devices, apparatus, files, programs, media, components, networks, systems, subsystems, and/or means, constituting any one or more of (a) a connection, whether direct or through one or more other devices, apparatus, files, programs, media, components, networks, systems, subsystems, or means, (b) a communications relationship, whether direct or through one or more other devices, apparatus, files, programs, media, components, networks, systems, subsystems, or means, and/or (c) a functional relationship in which the operation of any one or more devices, apparatus, files, programs, media, components, networks, systems, subsystems, or means depends, in whole or in part, on the operation of any one or more others thereof.

Referring now to the drawings, wherein like reference numerals designate corresponding structure throughout the views.

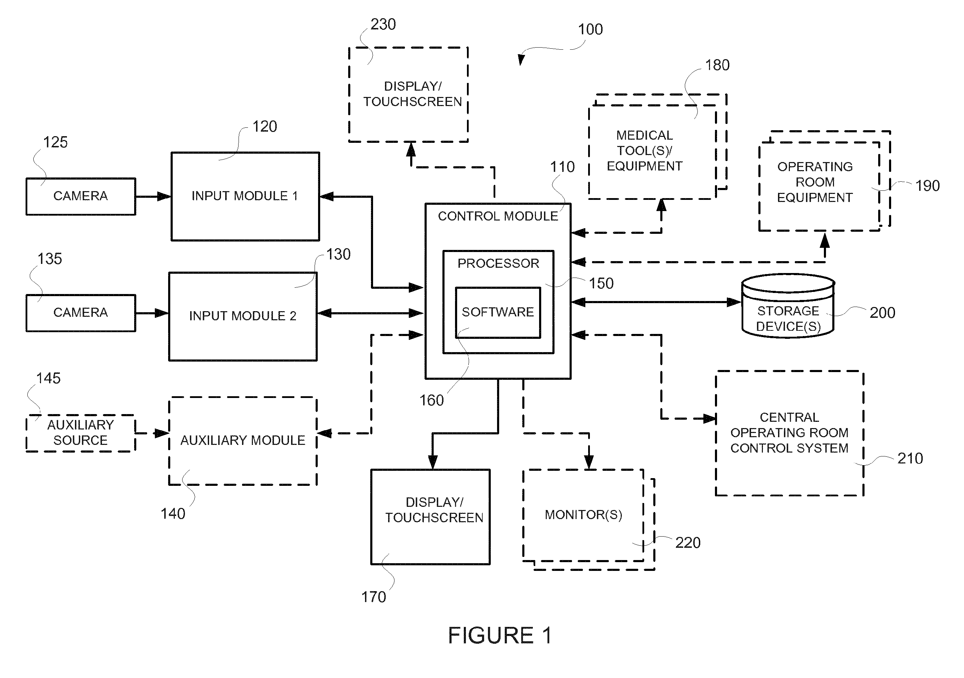

An advantageous embodiment of a medical imaging system of the present invention illustrated in FIG. 1. The medical imaging system 100 includes a control module 110. The control module is designed to accommodate general image processing and display functions for multiple camera types or families. These general functions include, for example, user interface, image capture and streaming functionality as well as input/output functionality for the display/monitor interfaces, system interface and control, and network connectivity. The control module 110 can be designed to accommodate one or multiple imaging modules.

In the embodiment shown in FIG. 1, the control module 110 is connected to a first input module 120 and a second input module 130. Each of the input modules 120, 130 is connected to an image source 125, 135, such as an endoscopic video camera. The input modules support all functions required for a group or family of image sources and provides compatibility between the family of image sources and the control module. The input modules 120, 130 include a processor for converting the image data received from the image sources 125, 135 into a format readable by the control module 110. The formatted image data is then transmitted to the control module 110, which processes the data into at least one output video signal.

At least one auxiliary input module 140 may optionally be connected to the control module 110. This auxiliary module supports one or more auxiliary sources 145, such as third party camera control units, C-Arm, X-Ray, ultrasound, personal computers and the like.

The input modules 120, 130 and the auxiliary module 140 are coupled to the control module 110 via a data transmission device, such as a cable, wireless, optical or any other suitable device known in the art. Once each of the input/auxiliary modules is connected to the control module 110, identifying information about each module is transmitted to the control module. The identifying information includes, but is not limited to, device settings, available operability commands, operational status of the device, and the like.

Connected to the control module 110 is display 170. In an advantageous embodiment, the display is a touchscreen that provides an interface for the user to control and interface with the control module 110 and various devices connected thereto. The control module 110 includes a processor 150 and a software 160 executing on the processor for presenting identifying information associated with each input module connected to the control module 110 on the display 170. The display 170 also displays the image data received by the control module 110 from one or more of the input modules 120, 130, 140.

The display/touchscreen 170 is positioned in the sterile environment and is accessible by, for example, a surgeon performing a procedure. The display/touchscreen 170 may be any suitable type of commercially available display or touchscreen device. The display/touchscreen is typically mounted on a boom or arm allowing the user to position the display/touchscreen 170 in a manner convenient for use, such as adjacent to or over the patient.

Furthermore, one or more medical tools and/or equipment 180 may be connected to the control module 110. Typically, medical and/or equipment 180 will be positioned in the sterile environment or in proximity thereto. Medical equipment will vary depending on the procedure being performed, and may include insufflations equipment, irrigation equipment, vacuum equipment and the like. Likewise, medical tool(s) may comprise a wide variety of medical tools used by the surgeon including, but not limited to catheterization devices, bi-polar cutting devices, lasers, rotating cutting devices, cell collection devices, suction devices and the like.

It is contemplated that medical tools and/or equipment 180 may be manufactured by different companies and therefore, the command and control signals for each of the medical tools/equipment may differ. As a particular medical tool/equipment is connected to the control module 110, the control module receives identifying information associated with the medial tool/equipment connected thereto and displays the information via the display/touchscreen 170, such that the medical tool/equipment 180 may be controlled by the user. Therefore, the control module 110 provides an interface between various differing types of signal formats such that the user may control a medical tool/equipment via the control module 110 if desired.

Operating room equipment 190 may also be connected to the control module 110. Operating room equipment 190 may comprise a wide variety of equipment that may be desirable to control by the surgeon or nurse including the operating room lights, the operating room blinds or shades, and the positioning of the operating room table. Operating room equipment 190 may also comprise hospital system including PACS, HIS and RIS, and remote image storage systems. As a piece of operation room equipment 190 is connected to the control module, the display 170 will present identifying information associated with the equipment to allow for user control of the equipment.

As shown in FIG. 1, storage device(s) 200 is also connected to the control module 110. Storage device(s) may comprise virtually any type of digital storage device including, solid state hard drive devices, magnetic hard drives devices, optical drive devices, removable storage devices and the like. For example, it may be desired to record a part or all of the procedure from the video cameras 125, 135 to a storage device 200 inserted into the control module. It may further be desired to save a part or all of the procedure to a hard drive device in the hospital information system for the hospitals records. Still further, the surgeon may desire to save a part or all of the procedure directly to a storage device on the surgeon's computer in the surgeon's office. There are many differing configurations that may be specified by the user either before or even during the procedure allowing for maximum system flexibility. It is also understood that the storage device(s) 200 may be connected to display 170 or any of the input modules.

Video monitor(s) 220 is also illustrated connected to the control module 110. The monitors 220 may comprise one or more surgical monitors positioned in the operating room. For example, a main surgical monitor is typically provided in the operating room, and often several surgical monitors are positioned at various locations in the operating room. Additionally, monitors positioned at remote locations may also be connected to the control module 110 to allow for video feeds to remote locations for telesurgery and teleconferencing such that a surgeon at a remote location could view the surgical procedure and provide input or comments to the surgeon performing the procedure. In addition, a video feed could be provided to a classroom environment for educational purposes so that medical students have the opportunity to see a particular medical procedure from a remote location.

The video feed from the input modules 120, 130 may be displayed on the monitor(s) 220. However, it is understood that the video feed from the input modules can also be displayed on the display 170 as desired.

In some embodiments, additional displays/touchscreens 230 may be provided. For example, a second touchscreen may be provided outside of the sterile environment, such as at a nurse's station. The second touchscreen 230 may be redundant to and provide all the functionality of the touchscreen 170. This way, a nurse has the ability to make adjustments based on the surgeon's direction if, for example, it is not convenient for the surgeon to do so on the touchscreen 170. It is also understood that additional features or a different configuration may be provided for the touchscreen 230. For example, while the touchscreen 170 may provide a video feed from the input modules 120, 130 in addition to various interfaces allowing control of the cameras 125,135 and various pieces of medical and operating room equipment and tools, it may not be necessary to provide the video feed to the touchscreen 230.

Additional touchscreens may be positions in or adjacent to the sterile environment and connected to the control module 110. For example, a separate touchscreen may be provided for an anesthesiologist to view the patient's vital signs, control the administration of anesthesia to the patient and to provide access to, for example, the hospital information system to pull up the patient's records and information.

The control module 110 may also be connected to a central operating room control system 210, which has a capability of controlling various medical devices, tools, and equipment inside an operating room. In one advantageous embodiment, the control module 110 may be connected to the central operating room control system 210 via a wireless network connection. The identifying information associated with various devices, tools and equipment connected to the control module 110 may be transmitted to the central operating room control system, 210 for display and control by a user. Alternatively, the image data received from the input modules 120, 130 is transmitted to the central operating room control system 210 for display, but the identifying data for the connected devices is displayed on the touchscreen 170.

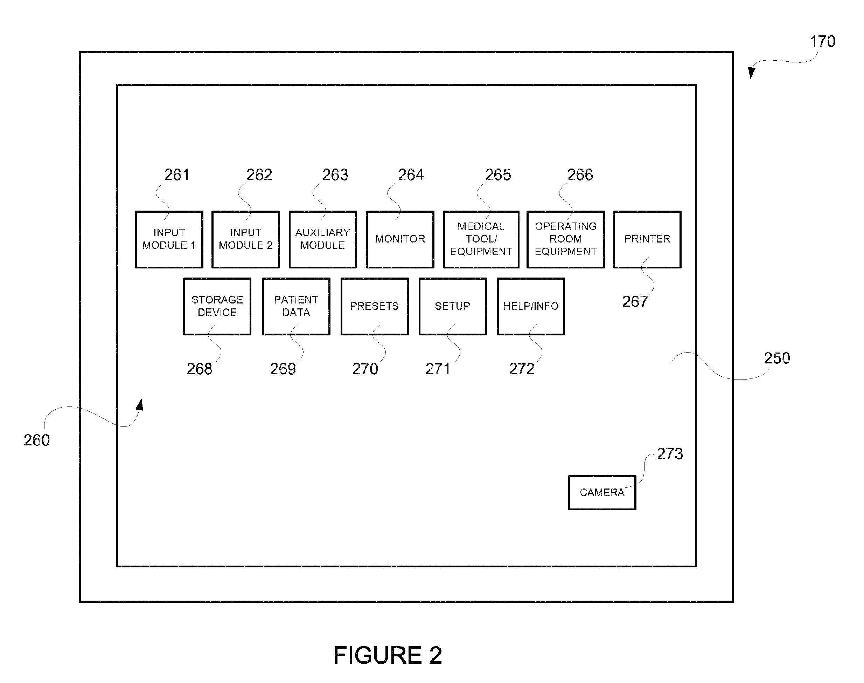

FIG. 2 illustrates an exemplary embodiment of the display/touchscreen 170. The touchscreen has a front panel 250 on which various data are displayed to the user. In the embodiment shown, a plurality of icons 260 are displayed associated with various devices connected to the control module. For example, the front panel 250 displays icons associated with the input modules connected to the control module 110, including a first input module icon 261, a second module input icon 262, and an auxiliary module icon 263. Once the input module icon is selected or activated by a user, the software 160 will automatically publish a list of available settings and corresponding commands for the selected input module. The available settings/commands may appear as a part of the icon itself, or may be presented as a separate control interface that is overlayed over the basic dashboard, as discussed in more detail below.

Additional icons associated with medical tool(s)/equipment 265 and operating room equipment 266 connected to the control module are also displayed. As discussed above, once the icons associated with the medical tool(s)/equipment or operating room equipment are selected, the available settings and commands for the corresponding device will be automatically presented on the display 170 by the software 160.

Furthermore, a number of icons associated with various available support devices are also displayed, including a storage device icon 268, a monitor icon 264, and a printer icon 267. The storage device icon 268 may be associated with one or more available storage devices, such as local or remote hard drives, or removable drive, coupled to the control module. Alternatively, a separate icon may be presented on the display for each of the available storage devices. Touching each of these icons will activate writing to each device. In additional embodiments, the system may be configured by the user such that video stream or any other data relating to a particular procedure is automatically saved to selected storage devices, but other storage locations only save the video stream when manually activated by the user.

The front panel also includes an icon associated with information about a particular patient that is undergoing the medical procedure. The touchscreen 170 may also display a setup icon 271 and a help/info icon 272.

The software 160 of the control module 110 is configurable based on a user's preferences and is updatable as various input modules and medical tools and equipment are coupled and/or decoupled from the control module 110. For example, selection and positioning of icons associated with image sources, medical tools/equipment, operating room equipment, supporting devices, etc., may be controlled by the surgeon's preferences.

It is understood that some, all or different icons may be presented on front panel 250 depending on the medical procedure to be performed and those indicated are only provided to indicate some of the types of devices/equipment that may be connected to the control module and used by the surgeon.

It is also understood that the configuration of the plurality of icons 260 shown in FIG. 2 is only exemplary, and that any other configuration may be employed in accordance with the present invention. For example, the icons may be arranged on the left, right, top or bottom portion of the front panel 250, or may be separated into groups and displayed in different sections of the display, depending on a user's preference. Furthermore, the appearance of the icons may also be selected based upon the user's preference.

For example, the user could set up the touchscreen such that a local hard drive, a remote hard drive and a removable storage device each have icons that appear on the touchscreen in a format based on the user's preferences. Touching each of these icons will activate writing to each device.

Alternatively, the user may only have one icon that is representative of multiple storage locations (e.g., local and remote hard drives) and a second icon for a removable drive such that the video stream is saved to the local and remote hard drives when selected but the removable drive is controlled separately. Still further, the system can be set up by the user as a preference to automatically save the entire video stream to selected storage devices but other storage locations only save the video stream when manually activated by the user.

It is contemplated that, when a particular icon presented on the display is activated, the icon visibly changes so as to indicate to the user that the device associated with the icon is activated. In some embodiments, the change to the icon includes a change in the color or appearance of the icon. It is further contemplated that any of the plurality of icons 260 can change during activation or interruption. For example, the input module icon may be changed to a "green" color when generating video data or to a "red" color when interrupted. Likewise, the storage icon can change color when data is being written to the storage device.

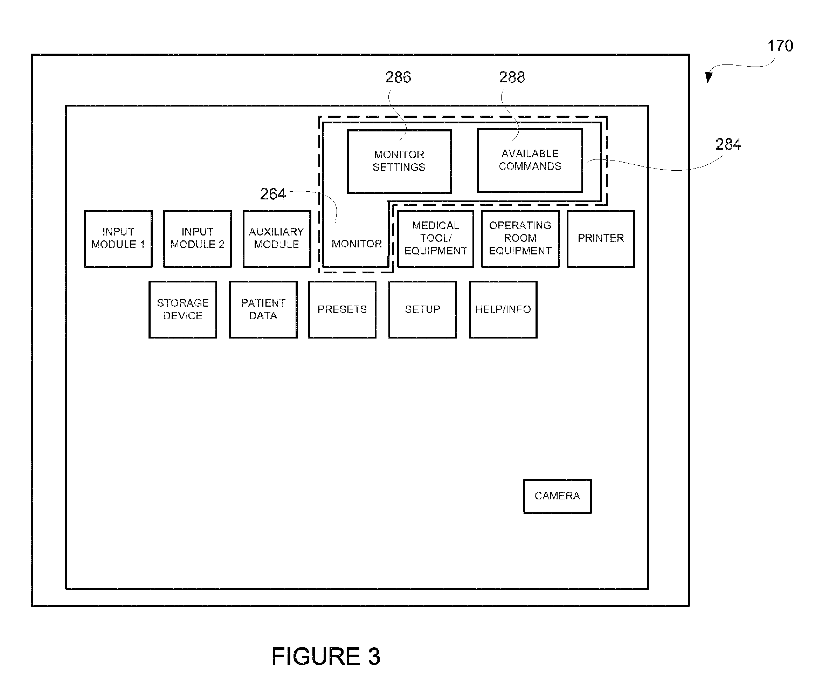

In other embodiments, the change to the icon includes a change in the configuration of the icon. For example, as illustrated in FIG. 3, when an icon, such as the monitor icon 264, is selected by a user, the icon changes its configuration. The dashed line shown around the monitor icon 264 illustrates that the icon is activated. The new configuration preferably includes a control interface 284 such that the user can change a setting of the device associated with the icon. The control interface 284 will automatically display settings 286 associated with the device represented by the icon, as well as user commands 288 available for the settings. For example, the monitor icon 264 may have a number of available settings, such as image(s) layout, image source(s), image flip, grid display option, etc. Each of these settings may have a plurality of commands executable by the user, such as turn on/off grid overlay, rotate image by certain number of degrees, select various available image sources, etc.

It is understood, however, that the embodiment shown in FIG. 3 is only exemplary, and that the appearance of the monitor icon 264 may be changed in other ways, such as by changing the icon's color or by changing the alpha-numeric or pictorial indication over the icon.

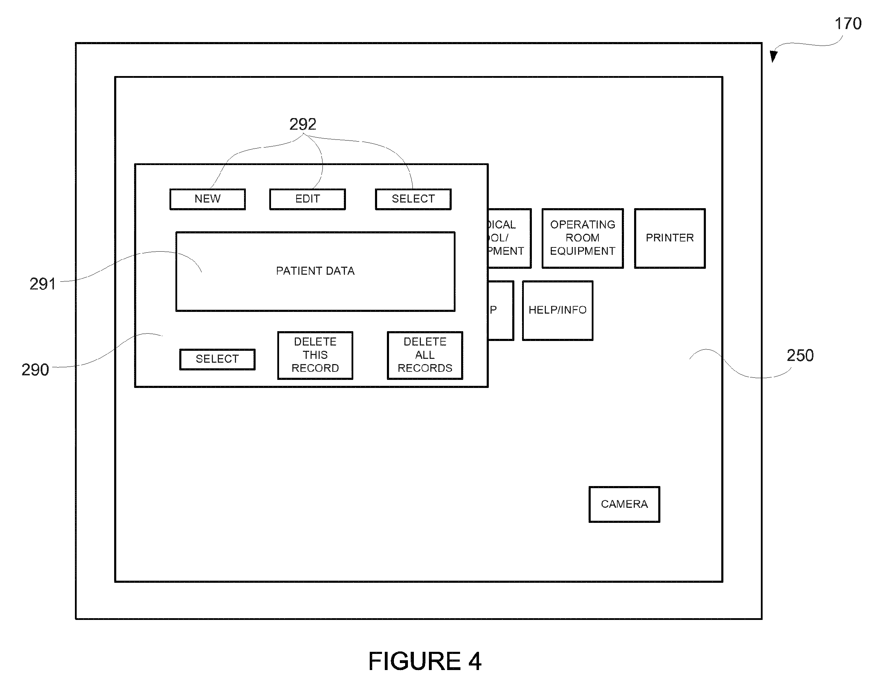

FIG. 4 illustrates another exemplary configuration of an icon selected by the user. In this case, the patient data icon 269 is selected and the configuration of the icon 269, as shown in FIG. 2, is changed to an expanded control interface 290 that is overlayed over other icons displayed on the front panel. This expanded configuration allows for more convenient entry of patient data by the user. The control interface 290 includes various patient data fields 291, such as patient's name, date of birth, ID number, physician name, procedure to be performed, etc. The control interface 290 also include available user commands 292, such as create a new record, select, edit or delete an existing record, etc. It is understood that the patient data icon may be associated with a storage device(s) provided in the control module 110, or any remote storage device or system, such as hospital information system, wherein patients' information may be stored.

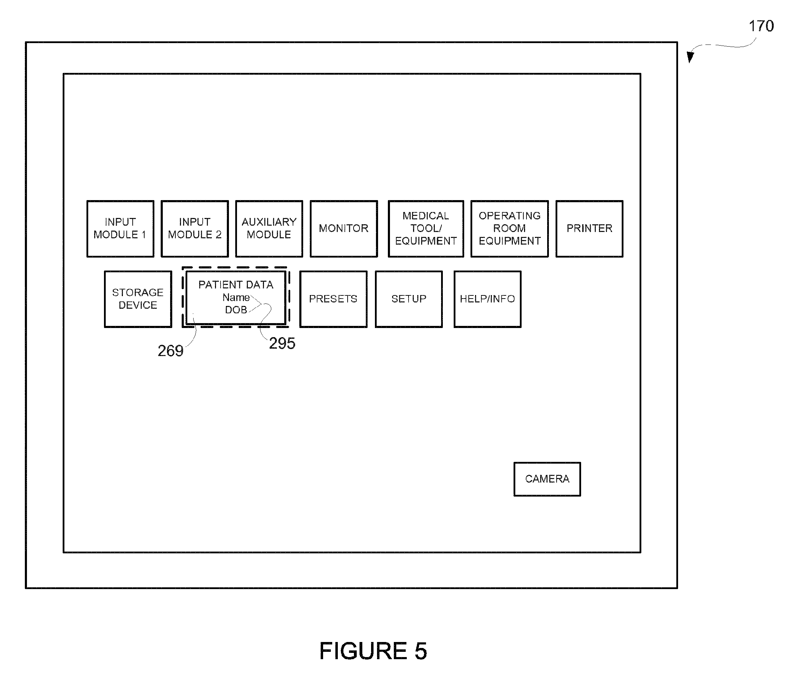

Once the user changes a particular setting, the control interface is removed from the display, and the changed setting is displayed on the icon associated with the device to which the setting was changed. For example, as shown in FIG. 5, once the control interface 290 is removed from display, the patient data icon 269 is changed to display certain information 295 associated with a particular patient, such as patient's name and date of birth. It is understood that the change to the icon may include any alpha-numeric or pictorial indication over the icon indicating a selected setting of the device associated with the icon.

In advantageous embodiments, setting indications on the icons are automatically updated as the setting of the device associated with the icon changes. For example, the storage device icon 268 may display a percentage of storage space available. As the amount of available storage space changes, the icon is automatically updated by software 160 to display the changed amount of storage space. The system may be configured to automatically check and update settings of devices connected to the control module in desired time intervals.

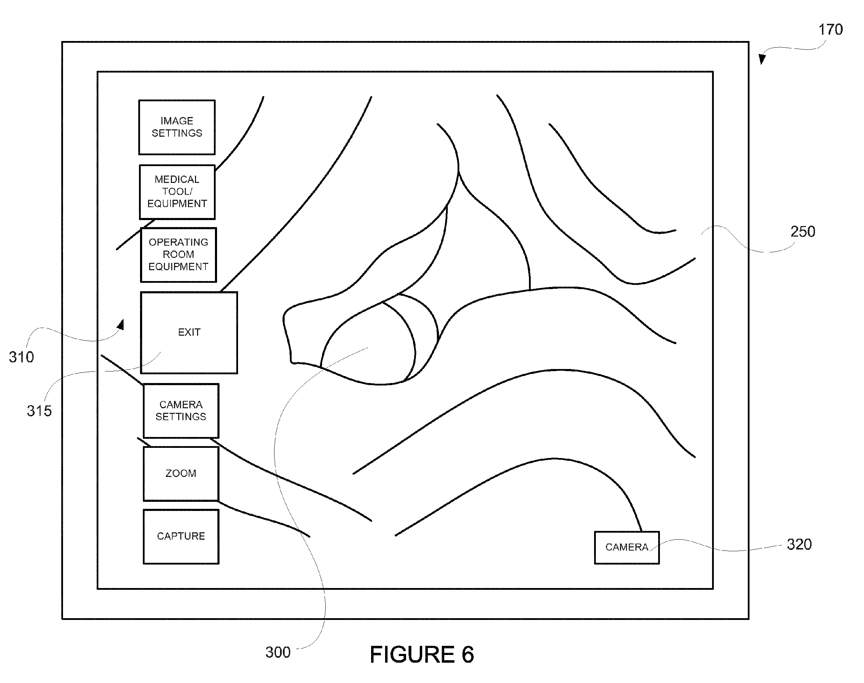

Once a camera head is connected to one of the input modules, the image data is transmitted to the control module and is displayed on the display 170. In one exemplary embodiment shown in FIG. 6, as soon as the camera head is connected, the basic dashboard as shown in FIGS. 1-5 extinguishes, and the image 300 received from the camera head is displayed on the front panel 250.

There is a separate icon 320 displayed on the front panel 250 representing the camera head connected to the control module 110. When the video output from the camera head is being streamed and shown on the display 170, the appearance of the icon 320 changes, for example by changing a color of the icon to "green", indicating that the video is being recorded. Once the video stream from the camera head is interrupted, the appearance of the icon 320 is changed again, such as by changing its color to "red", or the icon is removed from the display altogether, indicating that the video recording is off. It is understood that other ways of indicating that the camera head is operating may be utilized, including, for example, changing the configuration of the camera icon 320 or changing the alpha-numeric indication over the icon.

During the surgical procedure, a user manually brings up a plurality of icons 310 associated with various camera features and medical or support devices that are utilized to perform that procedure. The plurality of icons 310 may be a predetermined menu that is populated as a default when a user brings up the icons. For example, in the exemplary embodiment shown in FIG. 6, the default menu of icons 310 has the "exit" icon 315 active. It is understood that the control module 110 will receive identifying information about the input modules and other medical tool(s) and equipment connected thereto and will automatically populate icons associated with those devices, together with a list of settings and available user commands.

The default menu of icons may then be configured by the user based upon his or her preference. For example, some surgeons may prefer to use particular medical tools to perform a particular procedure, or may prefer certain camera or image settings. This way, the display of icons 310 is customized in accordance with the user's preference making it much more user friendly. In additional embodiments, a user may be able to bring up icons manually one by one based on the user's preference. It is understood that the icons may be brought to the display screen by a user's voice command instead of physical touching of the display. Alternatively, the icons may be populated via a second display/touchscreen that is positioned at a different location, such as a nurse's station. For example, the surgeon may instruct a nurse or other medical personnel which tools and device settings he or she would like to use for a particular procedure, such that icons associated with the desired tools and setting are activated by that individual via the second touchscreen positioned inside or outside of the sterile environment. The "exit" icon 315 may be activated at any time to remove the icon menu 310 from the screen such that only the video output 300 from the camera head is displayed on the front panel 250.

It should be noted that the embodiment illustrated in FIG. 6 is only exemplary. In other embodiments, once the camera head is connected to one of the input modules, a corresponding icon on the basic dashboard shown in FIGS. 1-5 may change its appearance, e.g. change color, configuration, or alpha-numeric indication over the icon, indicating to the user that the camera is connected to the system. The user may then activate the icon by touching the appropriate location on the front panel 250 of the touchscreen 170, or alternatively by a voice command. As a result, a video stream of images is shown on the display corresponding to a video output from the camera connected to the control module. The video stream may be shown in a separate window positioned anywhere on the front panel 250 of the display 170, or may be extended over the entire front panel 250 of the display similar to the embodiment shown in FIG. 6.

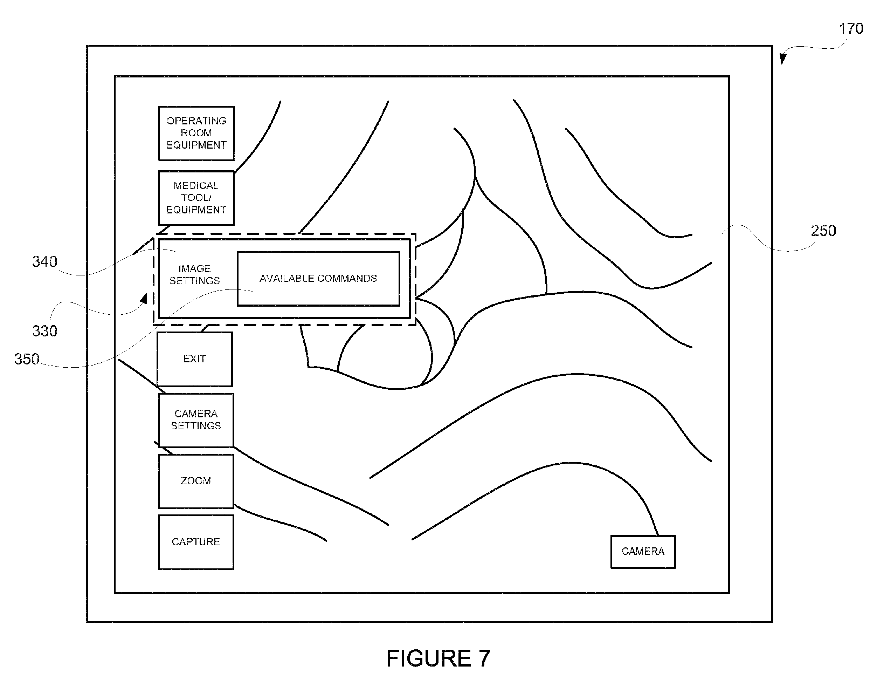

Once the plurality of icons 310 are populated on the front panel 250 of the display/touchscreen 170, the user may select any of the icons to adjust the setting of the device associated with the selected icon. FIG. 7 shows an image settings icon 330 activated by the user. The appearance of the icon 330 is changed into an expanded configuration indicating that the icon is selected. The dashed line shown around the icon 330 illustrates that the change it the appearance of the icon is not limited to the change in it configuration, but may be achieved by other means, such as changing the color or alphanumeric or pictorial indication over the icon.

As the icon 330 is selected, a list of available image settings 340 is populated, together with a list of executable commands 350 for each of the settings. Any type of command indications may be used in accordance with the present invention, depending on a type of command to be executed by a user. The available commands include, but are not limited to: activate commands for momentary activation of a particular setting, slider commands, e.g. to adjust exposure brightness level, and toggle commands, e.g. "blue light" mode on/off. Some types of commands may require knowledge of settings in the control module for optimal performance. In this case, the software 160 will present that information as part of the command control. For example, the edge enhancement setting in the input module may work best when the "zoom" setting in the control module is known. In such a case, the command is published with the setting range for the enhancement and the setting range for the zoom. In some embodiments, the input module is responsible for defining the operation of this interaction. In other embodiments, the interaction may be defined by the control module.

It is understood that the commands described above may be applicable to any of the icons displayed on the front panel 250 of the display 170. It is also understood that some of the commands may be executable by voice commands as opposed to touching the appropriate icons on the touchscreen 170.

As described above, the system of the present invention is completely configurable to allow the user to predefine or change/alter configurations to allow the user maximum freedom to control the imaging system 100 in a manner desired. For example, if one user prefers a particular layout and control setup, this has no bearing on the next user who may have a completely different layout and control setup. A particular user's preferred configuration may be saved on the control module 110, such that it can be later retrieved by that, or another, user, for example, by activating the presets icon 270 on the basic dashboard shown in FIG. 2.

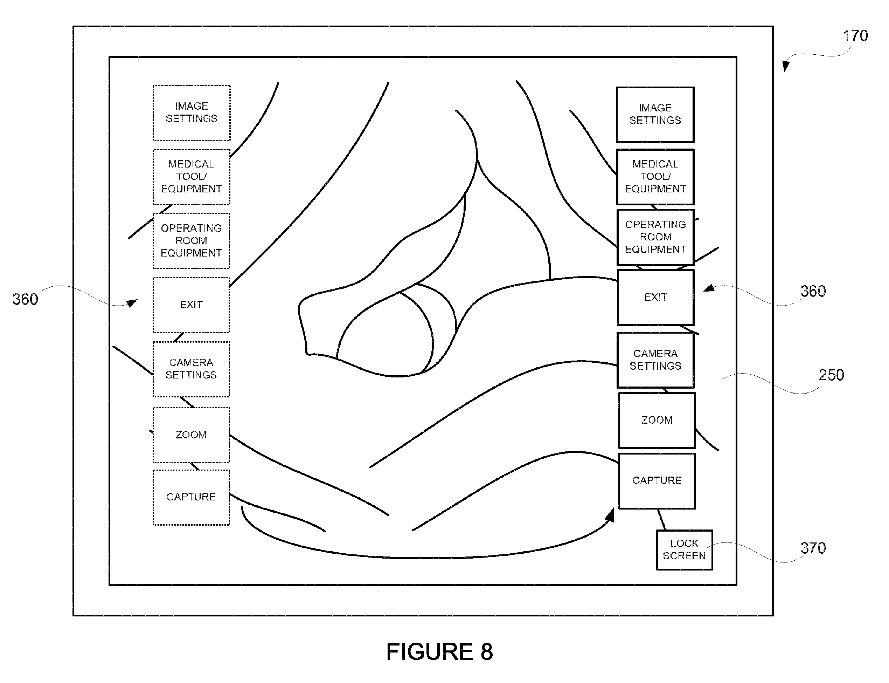

It is also understood that the display/touchscreen 170 could also be configured during a procedure if desired. For example, a particular user may be left-handed as opposed to right-handed and may prefer all controls to be displayed on the opposite side of the display 170. As shown in FIG. 8, the an icon 370 may be provided on the front panel 250 that allows the user to unlock the display/touchscreen 170 such that the icons 360 may be moved to a different portion on the display and/or rearranged into a different configuration. For example, as illustrated in FIG. 8, the icons 360 are moved from the left side of the display to the right side of the display. The icons 360 may be moved by touching them and then dragging them to the new location on the front panel 250.

Once a new desired location/configuration of the icons 360 is achieved, the lock screen icon 370 may be selected again to relock the touchscreen 170. In some advantageous embodiments, questions could be presented to the user to make sure they intended to "unlock" the display/touchscreen 170 prior to allowing the user to alter the current layout in the icons.

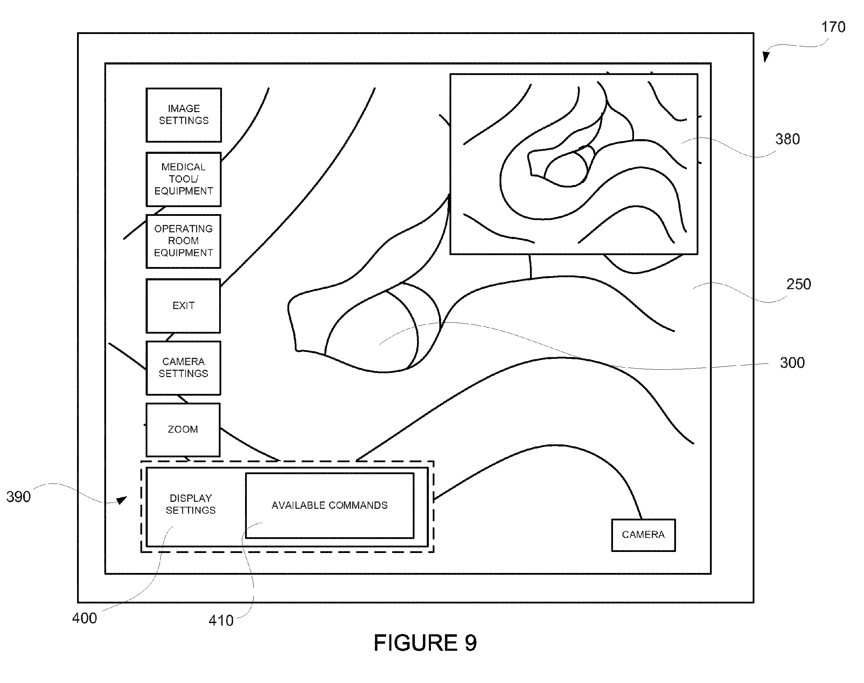

In addition to changing the configuration of icons of the front panel 250, a user is capable of changing the configuration of image data present on the display 170. For example, as shown in FIG. 9, in addition to the video stream 300 from the first camera head connected to the control module, the front panel 250 may display a separate window 380 showing different image data, also called as picture-in-picture display. Such image data may include a video stream from a second input module or an auxiliary module connected to the control module. Alternatively, the window 380 may show still images taken from the video stream 300 shown on the display. Furthermore, image data retrieved from a local or remote storage device may be shown in the window 380.

The positioning and configuration of the window 380 is also configurable by the user based on the user's preferences. As shown in FIG. 9, when the user activates the display icon 390, available display settings 400 are presented over the icon. Such display setting may include layout of the picture-in-picture display, source of the primary and secondary image data, option for swapping the primary and secondary image displays, and the like. The user may then select any available commands 410 associated with the settings 400 to configure the display 170.

It is understood that any of the features of the display/touchscreen 170 described above may also be provided with the secondary displays/touchscreens positioned at different locations inside or outside of the sterile environment. Additionally, the information displayed on the display/touchscreen 170 may also be displayed on one or more monitors connected to the control module, such as a surgical monitor positioned in the operating room or a monitor positioned at a remote location.

It is further noted that the configurations of the front panel 250 of the display 170 shown in the figures are only exemplary and that the user can do virtually anything with the data displayed on the front panel 250 to configure the touchscreen 170, 230 in a manner that is most desirable for the particular user. It is understood that icons, while shown as boxes in the figures, may instead be provided as a picture or image representative of the devices or settings associated with the icons. Likewise, the appearance of the icons to indicate that the icon has been selected by the user may be changed in numerous way. Furthermore, the "look" and "feel" of the icons themselves may be alterable by the user if desired. The icons may be changeable in numerous stages, for example, the icon for an irrigation pump may include a number superimposed over the icon to indicate the percentage of suction or may indicate a flow rate, and/or may change in color with the change in control. The goal of providing maximum configurability is to allow the user to configure the system to be as user friendly as possible to quickly and accurately provide information and control to the user to improve system operation and increase patient safety.

It is contemplated that all of this configurability can be accomplished prior to or during the procedure, or retrieved from stored preset configurations, such that the entire system is configured in accordance with the particular surgeon's configuration requirements simply by identification of the surgeon.

It is further important to note that not only the display/touchscreen 170, 230 as discussed above, but the entire system is configurable. For example, the data/information displayed on a surgical monitor or any other monitor connected to the control module can be automatically displayed, updated and adjusted as desired in the same manner as discussed above in connection with the touchscreen with some, all or completely different information being displayed on the monitor(s) as opposed to the touchscreen. Furthermore, additional displays/touchscreens may be connected to the control modules and may be independently configurable by software 160 or additional software executing on the control module processor.

Although the invention has been described with reference to a particular arrangement of parts, features and the like, these are not intended to exhaust all possible arrangements or features, and indeed many other modifications and variations will be ascertainable to those of skill in the art.

The present application dislcoses in particular the aspects defined in the following clauses which form part of the present description, but are not claims in accordance with decision J15/88 of the Legal Board of Appeal of the European Patent Office.

(1) A medical imaging system, comprising: a control module having a processor; at least one input module transmitting identifying information once connected to said control module; a display coupled to said control module for displaying image data received from said at least one input module; and a software executing on the processor for presenting icons on said display associated with the identifying information.

(2) The medical imaging system of clause 1, further comprising at least one camera connected to said at least one input module.

(3) The medical imaging system of clause 2, wherein said at least one camera includes an endoscope.

(4) The medical imaging system of clause 2, wherein said at least one input module includes a processor for converting video received from said at least one camera into a format readable by said control module.

(5) The medical imaging system of clause 4, wherein said control module processor processes formatted video into at least one output video signal.

(6) The medical imaging system of clause 1, wherein the display comprises a touchscreen displaying the icons and receiving actuation commands from a user.

(7) The medical imaging system of clause 1, wherein the identifying information presented on said display comprises a set of commands executable by said at least one input module.

(8) The medical imaging system of clause 1, wherein said software is configurable such that said icons and the image data are displayed on said display in a manner selected by a user.

(9) The medical imaging system of clause 1, wherein upon selection of an input module icon by a user, the icon displays a plurality of available commands associated with the selected input module.

(10) The medical imaging system of clause 9, wherein the plurality of available commands are configured and presented on said display based upon a user's defined configuration.

(11) The medical imaging system of clause 1, further comprising a monitor connectable to said control module displaying the image data received from the at least one input module.

(12) The medical imaging system of clause 11, wherein said software presents information associated with the monitor via said display.

(13) The medical imaging system of clause 11, wherein the image data is configured and presented on said monitor based upon a user's defined configuration.

(14) The medical imaging system of clause 1, further comprising a storage device coupled to said control module, wherein said software presents information associated with the storage device via said display.

(15) The medical imaging system of clause 1, wherein said at least one input module comprises a plurality of input modules, and wherein each input module has an icon associated therewith that is presented by said software via said display.

(16) The medical imaging system of clause 15, wherein the image data from the plurality of input modules is configured and presented on said display based upon a user's defined configuration

(17) The medical imaging system of clause 1, wherein when an icon presented on said display is activated, the icon visibly changes so as to indicate to the user that the device associated with the icon is activated.

(18) The medical imaging system of clause 17, wherein the change to the icon comprises a change in the color of the icon.

(19) The medical imaging system of clause 17, wherein the change to the icon comprises a change in the configuration of the icon.

(20) The medical imaging system of clause 17, wherein the change to the icon comprises an alpha-numeric indication over the icon indicating a setting of the device associated with the icon.

(21) The medical imaging system of clause 17, wherein when the icon is activated, a control interface is displayed on said display such that the user can change a setting of the device associated with the icon and upon changing the setting, the control interface is removed from said display and the changed setting is displayed on the icon associated with the device to which the setting was changed.

(22) The medical imaging system of clause 1, further comprising at least one medical tool coupled to said control module, wherein said software presents information associated with the at least one medical tool via said display.

(23) The medical imaging system of clause 1, further comprising at least one piece of operating room equipment coupled to said control module, wherein said at least one piece of operating room equipment has an icon associated therewith that is configured and presented on said display based upon a user's defined configuration.

(24) The medical imaging system of clause 1, further comprising an unlocking/locking mechanism presented on said display such that when the user activates the unlocking/locking mechanism, said display is selectively unlocked so that the user can manually adjust the positioning of the image data and icons on said display and upon activation of the unlocking/locking mechanism a second time, the position of the video output and icons on said display is locked.

(25) The medical imaging system of clause 24, wherein a particular user's preferred configuration of the image data and icons on said display is stored and retrievable by that or another user.

(26) The medical imaging system of clause 1, wherein said display comprises a first display and the system further comprises a second display positioned outside of the sterile environment, said second display coupled to said control module and providing all the functionality of said first display.

(27) The medical imaging system of clause 1, wherein said control module is connectable to central operating room control system via a network connection.

(28) The medical imaging system of clause 27, wherein the network connection is wireless.

There is disclosed a medical imaging system including a control module having a processor, at least one input module transmitting identifying information once connected to the control module, a display coupled to the control module for displaying image data received from the at least one input module, and a software executing on the processor for presenting icons on the display associated with the identifying information.

## Claims

1. Medical imaging system, comprising:
a control module having a processor;
at least one input module transmitting identifying information once connected to said control module;
a display coupled to said control module for displaying image data received from said at least one input module; and
a software executing on the processor for presenting icons on said display associated with the identifying information.

2. Medical imaging system of claim 1, further comprising at least one camera connected to said at least one input module.

3. Medical imaging system of claim 2, wherein said at least one input module includes a processor for converting video received from said at least one camera into a format readable by said control module, and wherein said control module processor processes formatted video into at least one output video signal.

4. Medical imaging system of any preceding claim, wherein the identifying information presented on said display comprises a set of commands executable by said at least one input module.

5. Medical imaging system of any preceding claim, wherein said software is configurable such that said icons and the image data are displayed on said display in a manner selected by a user.

6. Medical imaging system of any preceding claim, wherein upon selection of an input module icon by a user, the icon displays a plurality of available commands associated with the selected input module.

7. Medical imaging system of any preceding claim, further comprising a monitor connectable to said control module displaying the image data received from the at least one input module.

8. Medical imaging system of any preceding claim, further comprising a storage device coupled to said control module, wherein said software presents information associated with the storage device via said display.

9. Medical imaging system of any preceding claim, wherein when an icon presented on said display is activated, the icon visibly changes so as to indicate to the user that the device associated with the icon is activated.

10. Medical imaging system of claim 9, wherein when the icon is activated, a control interface is displayed on said display such that the user can change a setting of the device associated with the icon and upon changing the setting, the control interface is removed from said display and the changed setting is displayed on the icon associated with the device to which the setting was changed.

11. Medical imaging system of any preceding claim, further comprising at least one medical tool coupled to said control module, wherein said software presents information associated with the at least one medical tool via said display.

12. Medical imaging system of any preceding claim, further comprising at least one piece of operating room equipment coupled to said control module, wherein said at least one piece of operating room equipment has an icon associated therewith that is configured and presented on said display based upon a user's defined configuration.

13. Medical imaging system of any preceding claim, further comprising an unlocking/locking mechanism presented on said display such that when the user activates the unlocking/locking mechanism, said display is selectively unlocked so that the user can manually adjust the positioning of the image data and icons on said display and upon activation of the unlocking/locking mechanism a second time, the position of the video output and icons on said display is locked.

14. Medical imaging system of any preceding claim, wherein a particular user's preferred configuration of the image data and icons on said display is stored and retrievable by that or another user.

15. Medical imaging system of any preceding claim, wherein said control module is connectable to central operating room control system via a network connection.
